# EUROPÄISCHE PATENTANMELDUNG

(11) **EP 2 233 481 A1**
(43) Veröffentlichungstag der Anmeldung: **29.09.2010**
(21) Anmeldenummer: 09156382.5
(22) Anmeldetag: 27.03.2009
(51) Int. Cl.: C07D 401/06

(54) **Verfahren zur Herstellung von Mefloquin**

(71) Anmelder: Cilag Ltd., 8205 Schaffhausen (CH)
(72) Erfinder: Weigl, Ulrich, 78247, Hilzingen (DE); Sawicki, Marcin, 8600, Dübendorf (DE)
(74) Vertreter: Braun, André jr.

(57) **Zusammenfassung**

Verfahren zur Herstellung von erythro-alfa-2-Piperidyl-2,8-bis-(trifluormethyl)-4-chinolinmethanol (Mefloquin) mittels diastereo-selektiver Hydrierung von 2-Pyridyl-2,8-bis-(trifluormethyl)-4-chinolylketon (Dehydromefloquin) in einem geeigneten inerten Lösungsmittel und unter Verwendung von Edelmetallkatalysatoren, indem man (i) die Hydrierung von Dehydromefloquin in saurem Medium und (ii) in Gegenwart von mindestens einem katalytisch wirksamen Metall der Gruppe VIII des Periodischen Systems, sowie (iii) in Gegenwart von Bromidionen, durchgeführt wird; aus dem anschliessend isolierten Mefloquin als freie Base kann Mefloquin-Hydrochlorid hergestellt werden.

## Beschreibung

### Gegenstand der Erfindung

Die vorliegende Erfindung betrifft ein Verfahren zur Herstellung von Mefloquin mittels diastereo-selektiver Hydrierung von Dehydromefloquin.

### Stand der Technik

Die Verbindung Mefloquin entspricht der chemischen Bezeichnung erythro-alfa-2-Piperidyl-2,8-bis-(trifluormethyl)-4-chinolinmethanol, und ist ein bekannter Wirkstoff für die Behandlung der chloroquinresistenten Form von Malaria. Mefloquin wird zumeist als Mefloquin-Hydrochlorid, verwendet. Die Herstellung von Mefloquin ist an sich bekannt. Dabei wird in der Regel die Verbindung 2-Pyridyl-2,8-bis-(trifluormethyl)-4-chinolylketon (Dehydromefloquin) hydriert, wobei ein Racemat der pharmazeutisch wirksamen erythro-Form sowie der pharmazeutisch unwirksamen threo-Form erhalten wird. Dieses Racemat enthält die threo-Form in der Regel in einer Menge von bis zu 15 Gew.-%.

In EP 0 092 185 wird die Hydrierung von 2-Pyridyl-2,8-bis-(trifluormethyl)-4-chinolylketon (Dehydromefloquin) beschrieben, wobei die anschliessende Reinigung der erythro-Form dadurch erreicht wird, dass man das bei der Hydrierung anfallende Gemisch von erythro- und threo-alfa-2-Piperidyl-2,8-bis-(trifluormethyl)-4-chinolinmethanol-hydrochlorid mit wässrigem Methanol oder wässerigem Ethanol behandelt, wobei die threo-Form in Lösung geht und die erythro-Form auskristallisiert. Die Kristallisation des Hydrochlorid-Salzes aus einem Lösungsmittel, wie beispielsweise aus Methanol, zur Entfernung der unerwünschten threo-Diastereomers ist industriell nicht genügend effizient und führt zudem zu einer verminderten Gesamtausbeute. Es besteht deshalb der Bedarf für eine kosteneffiziente Synthese mit höherer Diastereoselektivität zugunsten der pharmazeutisch aktiven erythro-Form.

### Beschreibung der Erfindung

Es wurde nun gefunden, dass die Diastereoselektivität zugunsten von erythro-alfa-2-Piperidyl-2,8-bis-(trifluormethyl)-4-chinolinmethanol (Mefloquin) bei der Hydrierung von Dehydromefloquin deutlich erhöht wird, wenn diese in Gegenwart von Bromidionen erfolgt. Dies gilt insbesondere, wenn die Hydrierung in Anwesenheit von Bromidionen in saurem Medium und in Gegenwart von Edelmetallkatalysatoren durchgeführt wird, insbesondere in Gegenwart von Metallen der Gruppe VIII des Periodischen Systems, beispielsweise in Gegenwart von Rhodium, Platin oder Palladium oder Mischkatalysatoren, welche mehr als nur eines dieser Metalle enthalten. Die Diastereoselektivität zugunsten von erythro-alfa-2-Piperidyl-2,8-bis-(trifluormethyl)-4-chinolinmethanol wird insbesondere dann erhöht, wenn diese Metalle im Reaktionsgemisch in reiner Form vorliegen, wobei vorzugsweise reines Rhodium, gegebenenfalls in Kombination mit weiteren Metallen der Gruppe VIII des Periodischen Systems, eingesetzt wird.

Im weiteren wurde gefunden, dass bei der Hydrierung von Dehydromefloquin das erhaltene Reaktionsgemisch, vorzugsweise nach Filtration des Hydrierungskatalysators, direkt mit einem Hydroxid, beispielsweise mit Natriumhydroxid, versetzt werden kann, wobei Mefloquin als freie Base auskristallisiert. Dabei kristallisiert das Mefloquin in einer sowohl von Bromidionen als auch vom threo-Diastereomer gereinigten Form.

Durch anschliessende Umsetzung des derart erhaltenen Mefloquins mit Salzsäure wird Mefloquin-Hydrochlorid gebildet und kristallisiert aus, wobei Mefloquin-Hydrochlorid in einer deutlich höheren Gesamtausbeute erhalten wird.

Die vorliegende Erfindung ist in den Patentansprüchen definiert. Insbesondere betrifft die vorliegende Erfindung ein Verfahren zur Herstellung von erythro-alfa-2-Piperidyl-2,8-bis-(trifluormethyl)-4-chinolinmethanol (Mefloquin) mittels diastereo-selektiver Hydrierung von 2-Pyridyl-2,8-bis-(trifluormethyl)-4-chinolylketon (Dehydromefloquin) in einem geeigneten inerten Lösungsmittel und unter Verwendung von Edelmetallkatalysatoren, **dadurch gekennzeichnet, dass**
(i) die Hydrierung von Dehydromefloquin in saurem Medium
   und
(ii) in Gegenwart von mindestens einem katalytisch wirksamen
Metall der Gruppe VIII des Periodischen Systems, sowie (iii) in Gegenwart von Bromidionen, durchgeführt wird.

Die Erfindung betrifft im weiteren das erfindungsgemässe Verfahren, welches dadurch gekennzeichnet ist, dass man das bei der Hydrierung von Dehydromefloquin erhaltene Reaktionsgemisch, vorzugsweise nach Abfiltration des Hydrierungskatalysators, mit einer Base, vorzugsweise mit einem Hydroxid, vorzugsweise mit einem Alkalihydroxid, versetzt, und das gebildete Mefloquin als freie Base, gegebenenfalls nach Zugabe eines Antilösungsmittels, vorzugsweise durch Zugabe von Wasser, ausfällt bzw. auskristallisiert.

Die Erfindung betrifft im weiteren ein Verfahren zur Herstellung von Mefloquin-Hydrochlorid, welches dadurch gekennzeichnet ist, dass man Dehydromefloquin, in einem geeigneten inerten Lösungsmittel, unter Verwendung von mindestens einem katalytisch wirksamen Metall der Gruppe VIII des Periodischen Systems, sowie in Gegenwart von Bromidionen, unter den Bedingungen (i), (ii) und (iii), wie vorgehend beschrieben, hydriert; anschliessend das bei der Hydrierung von Dehydromefloquin erhaltene Reaktionsgemisch, vorzugsweise nach Abfiltration des Hydrierungskatalysators, mit einer Base, vorzugsweise mit einem Hydroxid, vorzugsweise mit einem Alkalihydroxid, versetzt, und das gebildete Mefloquin als freie Base, gegebenenfalls nach Zugabe eines Antilösungsmittels, vorzugsweise durch Zugabe von Wasser, ausfällt bzw. auskristallisiert; und anschliessend das derart ausgefällte bzw. auskristallisierte und gereinigte Mefloquin in an sich bekannter Weise in Mefloquin-Hydrochlorid umwandelt.

Die Erfindung betrifft im weiteren ein Verfahren zur Herstellung von Mefloquin, welches dadurch gekennzeichnet ist, dass man Dehydromefloquin, in einem geeigneten Lösungsmittel, unter Verwendung von mindestens einem katalytisch wirksamen Metall der Gruppe VIII des Periodischen Systems, sowie in Gegenwart von Bromidionen, unter den Bedingungen (i), (ii) und (iii), wie vorgehend beschrieben, hydriert; anschliessend das gebildete Mefloquin-Hydrobromid, vorzugsweise nach Abfiltration des Hydrierungskatalysators, aus dem Reaktionsgemisch isoliert; das derart erhaltene Mefloquin-Hydrobromid in einem geeigneten inerten Lösungsmittel löst, die Lösung mit einer Base, vorzugsweise mit einem Hydroxid, vorzugsweise mit einem Alkalihydroxid, versetzt, und gegebenenfalls nach Zugabe eines Antilösungsmittels, vorzugsweise durch Zugabe von Wasser, das gebildete Mefloquin als freie Base ausfällt bzw. auskristallisiert. Das derart erhaltene Mefloquin kann dann in an sich bekannter Weise in Mefloquin-Hydrochlorid umgewandelt werden.

Als geeignetes inertes Lösungsmittel für die Hydrierung und die weiteren Verfahrensschritte wird vorzugsweise ein mit Wasser mischbares Lösungsmittel verwendet, welches unter den Reaktionsbedingungen der erfindungsgemässen Hydrierung inert ist. Vorzugsweise ist dies ein alifatischer Alkohol, vorzugsweise ein alifatischer (C₁-C₈)-Alkohol, vorzugsweise Methanol, Ethanol, Propanol oder Butanol, vorzugsweise Methanol oder Ethanol oder ein Gemisch dieser Verbindungen.

Vorzugsweise enthält das inerte Lösungsmittel während der Hydrierungsreaktion höchstens 10 Gew.-% Wasser, vorzugsweise höchstens 5 Gew.-% Wasser, vorzugsweise höchstens 2 Gew.-% Wasser, und ist vorzugsweise im wesentlichen wasserfrei.

Die Kristallisation der freien Base aus dem geeigneten inerten Lösungsmittel, vorzugsweise einem alifatischen Alkohol, bzw. aus dem Reaktionsgemisch, erfolgt vorzugsweise durch Zugabe eines Antilösungsmittels, vorzugsweise durch Zugabe von Wasser. Dabei liegt das Verhältnis Lösungsmittel : Antilösungsmittel, bzw. Alkohol : Wasser, nach Zugabe der gesamten Menge des Antilösungsmittels vorzugsweise im Bereich von 2:1 bis 1:2, und vorzugsweise bei etwa 1:1.

Die Hydrierung erfolgt vorzugsweise in saurem Medium, wobei der saure Säurewert (pH-Wert) im Reaktionsgemisch vorzugsweise durch die Zugabe von Bromwasserstoff (HBr) als Bromidionenquelle erzeugt wird. Vorzugsweise liegt der Säurewert im Bereich von Null bis 5, vorzugsweise bei etwa 1. Dabei verwendet man Bromwasserstoff vorzugsweise als wässerige Lösung mit einer HBr-Konzentration von etwa 48 Gewichtsprozent.

Neben Bromwasserstoff (HBr) können auch Bromidsalze wie Natriumbromid (NaBr), Lithiumbromid (LiBr) und/oder Ammoniumbromid (NH₄Br) als Bromidionenquelle eingesetzt werden. Der saure pH-Wert kann durch Zugabe einer starken Säure erzeugt werden, beispielsweise durch Zugabe von Chlorwasserstoff (HCl), Bromwasserstoff (HBr), Schwefelsäure (H₂SO₄) und/oder Perchlorsäure (HClO₄), Trifluoressigsäure, para-Toluolsulfonsäure und verwandte Säuren, vorzugsweise durch Zugabe von Chlorwasserstoff. Dabei setzt man dem Reaktionsgemisch soviel Säure zu, bis der gewünschte Säurewert (pH-Wert) erreicht ist.

Die Konzentration der Bromidionen im Reaktionsgemisch beträgt vorzugsweise 0.5 bis 1.5 Equivalent (Atomgewicht) Bromid, vorzugsweise 0.9 bis 1.2 Equivalent Bromid, vorzugsweise 0.95 bis 1.1 Equivalent Bromid, und vorzugsweise 1.0 bis 1.1 Equivalent Bromid, pro Molgewicht der zu hydrierenden Menge Dehydromefloquin.

Die erfindungsgemässe Hydrierung wird in Gegenwart von mindestens einem katalytisch wirksamen Metall der Gruppe VIII des Periodischen Systems durchgeführt. Bevorzugte Metalle der Gruppe VIII des Periodischen Systems sind Nickel, Ruthenium, Rhodium, Iridium, Platin oder Palladium, vorzugsweise Rhodium, Iridium, Platin oder Palladium, vorzugsweise Rhodium, Platin oder Palladium, oder Mischkatalysatoren, welche mehr als nur eines dieser Metalle enthalten. Bevorzugt ist Rhodium, gegebenenfalls in Kombination mit einem weiteren Metall oder mit weiteren Metallen der Gruppe VIII des Periodischen Systems, vorzugsweise in Kombination mit Platin und/oder Palladium, wobei diese Metalle vorzugsweise in reiner Form vorliegen. Bevorzugt ist Rhodium.

Das verwendete katalytisch wirksame Metall oder die katalytisch wirksamen Metalle verwendet man vorzugsweise zusammen mit Kohlenstoff als Trägermaterial in an sich für Hydrierungsreaktionen üblichen und bekannten Mengen.

Für die Verwendung als Base zur Herstellung eines alkalischen Säurewertes sind Hydroxide, wie beispielsweise Alkalihydroxide, bevorzugt, insbesondere Natriumhydroxid oder Kaliumhydroxid, vorzugsweise Natriumhydroxid. Bei der Zugabe der Base wird vorzugsweise ein basischer Säurewert (pH-Wert) erzeugt, vorzugsweise ein pH-Wert im Bereich von 9 bis 14, vorzugsweise im Bereich von 10 bis 12 und vorzugsweise etwa 11.5.

Als Base können jedoch auch andere Verbindungen verwendet werden, wie beispielsweise Ammoniak, oder organische Amine, wie Triethylamin. Diese Verbindungen sind in der Regel teuerer und technisch schwieriger zu handhaben.

Die folgenden Beispiele erläutern die Erfindung ohne diese zu beschränken.

Allgemeine Prozessbeschreibung für Beispiel 1 und Beispiel 2: Dehydromefloquin wird in Methanol mit Bromwasserstoffsäure gemischt und in Gegenwart eines Katalysators unter Wasserstoffatmosphäre hydriert. Nach dem Ende der Hydrierung wird die Lösung filtriert, um den Katalysator zu entfernen. Die freie Base wird anschliessend durch Zusatz eines Überschusses von Natriumhydroxid freigesetzt, indem die resultierende Suspension der freien Base in der Hitze gelöst und durch Zugabe von Wasser kristallisiert wird. Mefloquin als freie Base wird filtriert und zu einer Lösung von Salzsäure in Essigsäure zugegeben. Die entstandene Suspension wird in der Hitze gelöst. Nach Zusatz von Wasser kristallisiert Mefloquin-Hydrochlorid aus. Das Produkt wird filtriert und im Vakuum getrocknet. In Beispiel 3 wird analog verfahren, jedoch wird Mefloquin-Hydrobromid als Zwischenprodukt isoliert.

### Beispiel 1

100 g Dehydromefloquin werden in 395 g Methanol mit 49.6 g einer Lösung von HBr (48%ig) in Wasser versetzt. Dann werden 5.0 g Pt [5% Pt auf Kohlenstoff (feucht)] zugesetzt und unter 2.5-3.5 bar Wasserstoffdruck, bei 33-37°C, bis zum Verbrauch von 1.08 mol (4 eq.) Wasserstoff, hydriert. Die Reaktionsmischung wird unter Stickstoff durch 10 g Celite filtriert und der Filterkuchen zweimal mit je 80 g Methanol nachgewaschen. Der pH-Wert des filtrierten Reaktionsgemisches wird mit Natronlauge (30%) auf pH = 11.5 eingestellt und die entstandene Suspension auf 65-75°C erwärmt, wobei eine Lösung erhalten wird. Nach Zugabe von 400 g Wasser kristallisiert Mefloquin als freie Base. Die erhaltene Suspension wird auf Raumtemperatur gekühlt und filtriert. Der Filterkuchen wird mit 90 g einer Mischung von MeOH:H₂O von 1:1 und anschliessend mit 100 g Wasser gewaschen. Mefloquin freie Base wird dann in einer Lösung von 25.5 g Salzsäure (32%ig) und 67.0 g Wasser suspendiert und mit 168.0 g Essigsäure versetzt. Die Suspension wird auf 90-95°C erwärmt, wobei eine Lösung erhalten wird. Nach Zugabe von 487 g Wasser kristallisiert Mefloquin-Hydrochlorid aus. Die entstandene Suspension wird auf 0-5°C gekühlt und mit 25.5 g Salzsäure (32%ig) versetzt. Mefloquin-Hydrochlorid wird filtriert und zweimal mit jeweils 100g kaltem Wasser gewaschen. Die Kristalle werden bei 140°C im Vakuum getrocknet. Ausbeute: 88.7 g (entspr. 80%).

### Beispiel 2

20 g Dehydromefloquin werden in 79.0 g Methanol mit 9.8 g einer 48%igen Lösung von Bromwasserstoff (HBr) in Wasser, versetzt.
0.5 g des Katalysators, enthaltend Rhodium und Platin (10% Rh, 1% Pd, auf Kohlenstoffträger) werden hinzugesetzt. Es wird unter 2.5-3.5 bar Wasserstoffdruck, bei 33-37°C, bis zum Verbrauch von 0.22 mol (4 eq.) Wasserstoff hydriert. Die Reaktionsmischung wird unter Stickstoff durch 10 g Celite filtriert und zweimal mit jeweils 16 g Methanol gewaschen. Der pH-Wert wird mit wässeriger Natronlauge (30%ig) auf pH = 11.5 eingestellt und die entstehende Suspension auf 65-75°C erwärmt, wobei eine Lösung erhalten wird. Mefloquin als freie Base kristallisiert nach Zugabe von 80 g Wasser aus. Die Suspension wird auf Raumtemperatur gekühlt und filtriert. Der Filterkuchen wird mit 18g einer Mischung MeOH:H₂O von 1:1 und anschliessend mit 20 g Wasser gewaschen. Mefloquin freie Base wird dann in einer Lösung von 5.92 g wässerige Salzsäure (32%ig) und 43.5 g Methanol gelöst. Der Suspension wird auf Rückflusstemperatur erwärmt. Nach Zugabe von 345 g Wasser kristallisiert Mefloquin-Hydrochlorid aus. Die Suspension wird auf 0-5°C gekühlt, filtriert und zweimal mit je 20 g kaltem Wasser gewaschen. Die Kristalle werden bei 100°C in Vakuum getrocknet.

Ausbeute: 18.2 g, entsprechend 80%.

### Beispiel 3

100 g Dehydromefloquin werden in 395 g Methanol mit 49.6 g einer wässerigen Lösung von Bromwasserstoff (HBr, 48%ig) versetzt. Dann werden 5.8 g Katalysator [Pd 4% - Rh 1% auf Kohlenstoffträger (feucht)] zugesetzt. Es wird unter 2.5-3.5 bar Wasserstoffdruck bei 43-47°C bis zum Verbrauch von 1.08 mol (4 eq.) Wasserstoff hydriert. Die Reaktionsmischung wird unter Stickstoff durch 10 g Celite filtriert und zweimal mit jeweils 80 g Methanol gewaschen. Von der Lösung werden 350 g Methanol abdestilliert. Zur verbleibenden heissen Lösung (65-70°C) werden 800 g Wasser langsam zugegeben. Die entstandene Suspension wird dann auf Raumtemperatur (20-25°C) gekühlt und filtriert. Der Filterkuchen wird mit zweimal je 50 g kaltem Wasser gewaschen. Das erhaltene Mefloquin-Hydrobromid wird in 306 g Methanol gelöst und der pH-Wert mit Natronlauge (30%ig) auf pH = 11.5 eingestellt. Die entstandene Suspension wird bei 65-75°C gelöst und Mefloquin freie Base durch Zusatz von 388 g Wasser kristallisiert. Die Suspension wird auf Raumtemperatur gekühlt und filtriert. Der Rückstand wird mit 90g einer Mischung MeOH:H₂O von 1:1 und anschliessend mit 100 g Wasser gewaschen. Mefloquin freie Base wird in einer Lösung von 22.7 g Salzsäure (32%ig) in 60.0 g Wasser suspendiert und mit 155.0 g Essigsäure versetzt. Die Suspension geht beim Erwärmen auf 90-95°C in Lösung und Mefloquin-Hydrochlorid wird durch Zugabe von 433 g Wasser kristallisiert. Die Suspension wird auf 0-5°C gekühlt und mit 22.7 g wässeriger Salzsäure (32%ig) versetzt. Mefloquin-Hydrochlorid wird filtriert und mit 2 x 100g kalten Wasser nachgewaschen. Die Kristalle werden bei 140°C im Vakuum getrocknet. Ausbeute: 81.5 g (71%).

### Beispiel 4

Mefloquin-Hydrochlorid wird analog zu den Beispielen 1 bis 3 hergestellt. Dabei werden der Katalysator und die Reaktionsbedingungen variiert. Auch wird Zugabe von Bromwasserstoff (HBr) mit der Zugabe von Chlorwasserstoff (HCl) verglichen. Die Resultate sind in Tabelle 1 dargestellt.

Die Resultate zeigen, dass die Bromidionen überraschenderweise deutlich aktiver sind als die Chloridionen, welche (im Gegensatz zu Bromid) keine messbare Selektivitätssteigerung ergeben. Von Bedeutung für die vorliegende Erfindung ist zudem, dass die Kristallisation Mefloquin als freie Base ergibt. Dadurch wird die einfache Entfernung der eingesetzten Salze ermöglicht sowie auch die Effizienz der Reinigung des Produktes gesteigert (deutlich weniger Produktverlust in die Mutterlage im Vergleich zur bekannten Aufreinigung über eine Kristallisation als Mefloquin-Hydrochlorid).

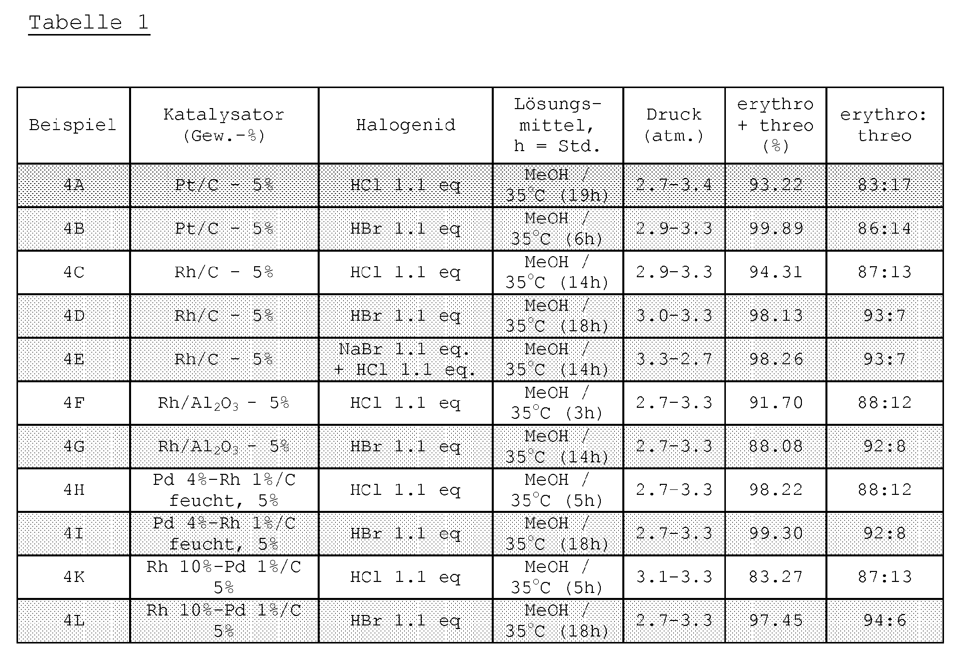

## Patentansprüche

1. Verfahren zur Herstellung von erythro-alfa-2-Piperidyl-2,8-bis-(trifluormethyl)-4-chinolinmethanol (Mefloquin) mittels diastereo-selektiver Hydrierung von 2-Pyridyl-2,8-bis-(trifluormethyl)-4-chinolylketon (Dehydromefloquin) in einem geeigneten inerten Lösungsmittel und unter Verwendung von Edelmetallkatalysatoren, **dadurch gekennzeichnet, dass**
(i) die Hydrierung von Dehydromefloquin in saurem Medium
und
(ii) in Gegenwart von mindestens einem katalytisch wirksamen Metall der Gruppe VIII des Periodischen Systems, sowie
(iii) in Gegenwart von Bromidionen, durchgeführt wird.

2. Verfahren nach Anspruch 1, **dadurch gekennzeichnet, dass** das Lösungsmittel mit Wasser mischbar und unter den Reaktionsbedingungen der Hydrierung inert ist.

3. Verfahren nach Anspruch 1 oder 2, **dadurch gekennzeichnet, dass** als Lösungsmittel ein alifatischer Alkohol, vorzugsweise ein alifatischer (C₁-C₈)-Alkohol, vorzugsweise Methanol, Ethanol, Propanol oder Butanol, vorzugsweise Methanol oder Ethanol oder ein Gemisch dieser Verbindungen verwendet wird.

4. Verfahren nach einem der Ansprüche 1-3, **dadurch gekennzeichnet, dass** das Lösungsmittel während der Hydrierungsreaktion höchstens 10 Gew.-% Wasser, vorzugsweise höchstens 5 Gew.-% Wasser, vorzugsweise höchstens 2 Gew.-% Wasser, und vorzugsweise im wesentlichen wasserfrei ist.

5. Verfahren nach einem der Ansprüche 1-4, **dadurch gekennzeichnet, dass** der saure pH-Wert im Reaktionsgemisch durch die Zugabe von Bromwasserstoff erzeugt wird.

6. Verfahren nach einem der Ansprüche 1-5, **dadurch gekennzeichnet, dass** der pH-Wert im Bereich von Null bis 5, vorzugsweise bei etwa 1 liegt.

7. Verfahren nach einem der Ansprüche 1-6, **dadurch gekennzeichnet, dass** als Bromidionenquelle ein Bromidsalz, vorzugsweise Natriumbromid, Lithiumbromid und/oder Ammoniumbromid eingesetzt und der saure pH-Wert durch Zugabe einer starken Säure erzeugt wird, vorzugsweise durch Zugabe von Chlorwasserstoff, Bromwasserstoff, Schwefelsäure, Perchlorsäure, Trifluoressigsäure und/oder para-Toluolsulfonsäure, vorzugsweise durch Zugabe von Chlorwasserstoff.

8. Verfahren nach einem der Ansprüche 1-7, **dadurch gekennzeichnet, dass** die Konzentration der Bromidionen im Reaktionsgemisch 0.5 bis 1.5 Equivalent Bromid, vorzugsweise 0.9 bis 1.2 Equivalent Bromid, vorzugsweise 0.95 bis 1.1 Equivalent Bromid, und vorzugsweise 1.0 bis 1.1 Equivalent Bromid, pro Molgewicht der zu hydrierenden Menge Dehydromefloquin beträgt.

9. Verfahren nach einem der Ansprüche 1-8, **dadurch gekennzeichnet, dass** die Hydrierung in Gegenwart von Nickel, Ruthenium, Rhodium, Iridium, Platin oder Palladium, vorzugsweise in Gegenwart von Rhodium, Iridium, Platin oder Palladium, vorzugsweise Rhodium, Platin oder Palladium, oder in Gegenwart von Mischkatalysatoren, welche mehr als nur eines dieser Metalle enthalten, durchgeführt wird.

10. Verfahren nach Anspruch 9, **dadurch gekennzeichnet, dass** die Hydrierung in Gegenwart von Rhodium, gegebenenfalls in Kombination mit einem weiteren Metall oder mit weiteren Metallen der Gruppe VIII des Periodischen Systems, vorzugsweise in Kombination mit Platin und/oder Palladium, durchgeführt wird und diese Metalle vorzugsweise in reiner Form vorliegen.

11. Verfahren nach einem der Ansprüche 1-10, **dadurch gekennzeichnet, dass** man das bei der Hydrierung erhaltene Reaktionsgemisch, vorzugsweise nach Abfiltration des Hydrierungskatalysators, mit einer Base, vorzugsweise mit einem Hydroxid, versetzt, und das gebildete Mefloquin als freie Base auskristallisiert.

12. Verfahren nach Anspruch 11, **dadurch gekennzeichnet, dass** die Kristallisation der freien Base aus dem Reaktionsgemisch durch Zugabe eines Antilösungsmittels, vorzugsweise durch Zugabe von Wasser erfolgt, wobei das Verhältnis Lösungsmittel zu Antilösungsmittel, vorzugsweise Alkohol zu Wasser, nach Zugabe der gesamten Menge des Antilösungsmittels, im Bereich von 2:1 bis 1:2, und vorzugsweise bei etwa 1:1 liegt.

13. Verfahren nach Anspruch 11 oder 12, **dadurch gekennzeichnet, dass** als Base zur Herstellung des alkalischen Säurewertes ein Hydroxid, vorzugsweise ein Alkalihydroxid, vorzugsweise Natriumhydroxid oder Kaliumhydroxid, vorzugsweise Natriumhydroxid verwendet wird, wobei ein Säurewert im Bereich von 9 bis 14, vorzugsweise im Bereich von 10 bis 12, und vorzugsweise etwa 11.5, erzeugt wird.

14. Verfahren nach einem der Ansprüche 11-13, **dadurch gekennzeichnet, dass** man das als freie Base auskristallisierte Mefloquin in an sich bekannter Weise in Mefloquin-Hydrochlorid umwandelt.

15. Verfahren nach einem der Ansprüche 1-10, **dadurch gekennzeichnet, dass** man das gebildete Mefloquin-Hydrobromid, vorzugsweise nach Abfiltration des Hydrierungskatalysators, aus dem Reaktionsgemisch isoliert; das derart erhaltene Mefloquin-Hydrobromid in einem geeigneten inerten Lösungsmittel löst, die Lösung mit einer Base, vorzugsweise mit einem Hydroxid, vorzugsweise mit einem Alkalihydroxid, versetzt, und gegebenenfalls nach Zugabe eines Antilösungsmittels, vorzugsweise durch Zugabe von Wasser, das gebildete Mefloquin als freie Base auskristallisiert.

16. Verfahren nach Anspruch 15, **dadurch gekennzeichnet, dass** man das erhaltene Mefloquin in an sich bekannter Weise in Mefloquin-Hydrochlorid umwandelt.
